# EUROPÄISCHE PATENTSCHRIFT

(11) **EP 0 959 137 B1**
(45) Veröffentlichungstag und Bekanntmachung des Hinweises auf die Patenterteilung: **02.01.2002**
(21) Anmeldenummer: 99108948.3
(22) Anmeldetag: 05.05.1999
(51) Int. Cl.: C12P 13/20, C07K 5/06

(54) **Verfahren zur Herstellung von L-Asparaginsäure**
Process for the preparation of aspartic acid
Procédé de préparation d'acide aspartique

(30) Priorität: 22.05.1998 AT 87898
(43) Veröffentlichungstag der Anmeldung: 24.11.1999
(73) Patentinhaber: DSM Fine Chemicals Austria Nfg GmbH & Co KG, 4021 Linz (AT)
(72) Erfinder: Giselbrecht, Karl-Heinz, 4061 Pasching (AT); Schaller, Josef, 4020 Linz (AT)
(74) Vertreter: Klostermann, Ingrid

(56) Entgegenhaltungen:
- EP-A- 0 612 784
- EP-A- 0 613 920
- EP-A- 0 832 982
- EP-A- 0 945 517
- DE-A- 3 029 348
- FR-A- 2 158 330
- US-A- 3 214 345
- US-A- 4 560 653
- PATENT ABSTRACTS OF JAPAN vol. 1996, no. 06, 28. Juni 1996 (1996-06-28) & JP 08 033493 A (NIPPON SHOKUBAI CO LTD), 6. Februar 1996 (1996-02-06)

## Beschreibung

L-Asparaginsäure ist ein essentieller Ausgangsstoff für verschiedenste Additive für die pharmazeutische Industrie und den Nahrungsmittelsektor. Beispielsweise wird L-Asparaginsäure zur Herstellung von künstlichem Süßstoff, wie etwa Aspartam, eingesetzt. Zur Herstellung von L-Asparaginsäure sind deshalb bereits eine Vielzahl von chemischen und enzymatischen Verfahren beschrieben. Bei den enzymatischen Varianten wird L-Asparaginsäure zumeist durch enzymatische Addition von Ammoniak an Fumarsäure mit nachfolgender Ausfällung aus der so erhaltenen L-Ammoniumaspartat-Lösung gewonnen.
Gemäß dem Stand der Technik kann das Ausfällen von L-Asparaginsäure beispielsweise durch Zugabe von Schwefelsäure oder Salzsäure oder anderer Säuren, wie etwa p-Toluolsulfonsäure, erfolgen.
Die Nachteile dabei sind jedoch, daß der Verlust an Ammoniak groß ist und eine große Menge an Abwasser mit einer hohen Konzentration an Ammoniumsalzen der verwendeten Säuren ausgeschieden wird.
Aus diesem Grund wurde versucht, Möglichkeiten zu finden, die Abwasserproblematik zu verringern oder gänzlich zu vermeiden.

Gemäß US 4,560,653 erfolgt das Ausfallen der L-Asparaginsäure beispielsweise durch Zugabe von Maleinsäure. Nach der Abtrennung von L-Asparaginsäure wird die verbleibende Mutterlauge einem Isomerisierungsschritt unterworfen, bei dem Maleinsäure in Fumarsäure etwa mittels eines Bromionen enthaltenden Katalysators isomerisiert wird, anschließend gereinigt und der enzymatischen Reaktion erneut zugeführt wird.
Um den Isomerisierungsschritt zu umgehen, wurde nach weiteren geeigneten Zusätzen zur Ausfällung von L-Asparaginsäure gesucht In der japanischen Offenlegungsschrift JP 08-33493 (Chem. Abstracts 124: 315 167) wird die Verwendung von Fumarsaure bzw Fumarsauresalz als Fallungsmittel beschrieben. Der Nachteil dieser Verfahrensvariante liegt in der schlechten Wasserlöslichkeit von Fumarsäure, wodurch bei der Aufarbeitung der Mutterlauge entweder große Mengen an Wasser abdestilliert werden müssen oder eine sehr verdünnte Fahrweise mit großen Reaktionsvolumen erforderlich ist.

Aufgabe der Erfindung war es demnach ein Verfahren zu finden, das die bisherigen Probleme bei der Ausfällung von L-Asparaginsäure vermeidet und das zu L-Asparaginsäure in hohen Ausbeuten und hoher Reinheit führt.
Unerwarteterweise konnte diese Aufgabe durch die Verwendung von Salpetersäure als Fällungsmittel und anschließender Nanofiltration der Mutterlauge gelöst werden.

Gegenstand der Erfindung ist demnach ein Verfahren zur Herstellung von L-Asparaginsäure durch enzymkatalysierte Umsetzung von Fumarsäure mit Ammoniak zu Ammonium-L-Aspartat in einem inerten Verdünnungsmittel aus dem nachfolgend die L-Asparaginsäure durch Zusatz einer Säure ausgefällt wird, das dadurch gekennzeichnet ist, daß
a) Fumarsäure in einem inerten Verdünnungsmittel in Gegenwart von Aspartase oder Aspartase-produzierenden Mikroorganismen mit Ammoniak zu Ammonium-L-Aspartat umgesetzt,
b) gegebenenfalls überschüssiger Ammoniak aus dem Reaktionsgemisch entfernt und
c) L-Asparaginsäure durch Zugabe von Salpetersäure ausgefällt, aus dem Reaktionsgemisch abgetrennt, gewaschen und getrocknet wird, anschließend
d) aus dem Fällungsfiltrat mittels Nanofiltration Rest-L-Asparaginsäure und Restfumarsäure abgetrennt, das Retentat in Schritt c) rückgeführt wird und
e) das Filtrat aus Schritt d) aufkonzentriert, das dabei anfallende Destillat mit dem in Schritt b) gegebenenfalls abgetrennten Ammoniaküberschuß in Schritt a) wieder zusetzt und gegebenenfalls das aufkonzentrierte Filtrat als Wertstoff für Stickstoffdünger verwendet wird

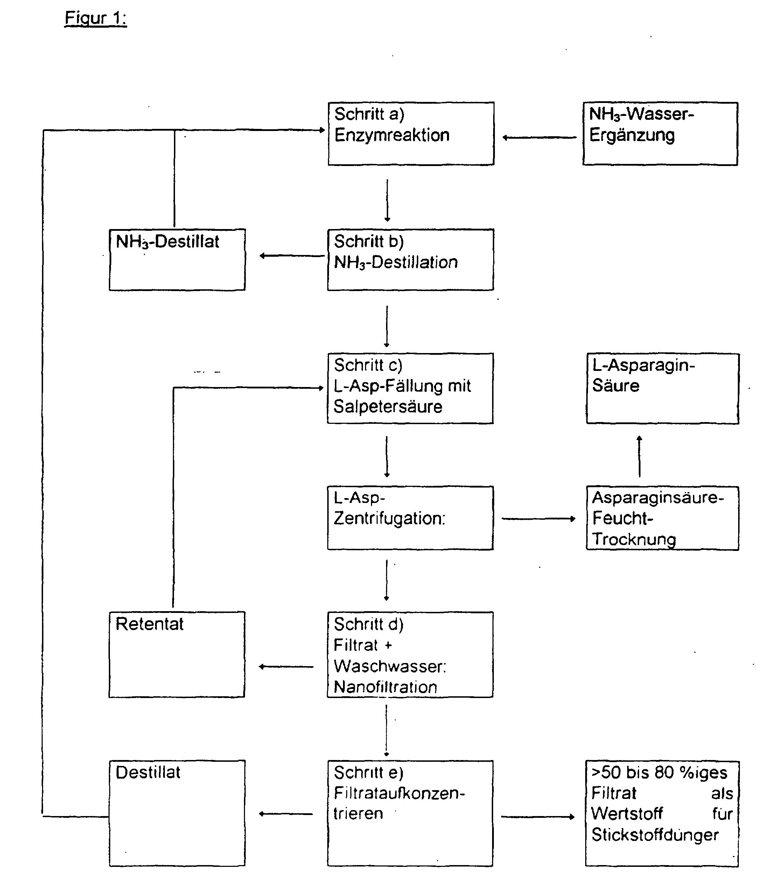

Bei dem erfindungsgemäßen Verfahren wird L-Asparaginsäure mittels Salpetersäure ausgefällt. Die Reaktionsströme werden erfindungsgemäß im Kreis gefahren. Das erfindungsgemäße Verfahren ist aus Abbildung 1 ersichtlich, in der die Reaktionsströme schematisch dargestellt sind.
Das in Schritt e) erhaltene aufkonzentrierte Filtrat kann direkt als Wertstoff für Stickstoffdünger verwendet werden.
Der erste Schritt des erfindungsgemäßen Verfahrens beinhaltet die enzymatische Reaktion der Fumarsäure mit Ammoniak. Die Reaktion findet dabei in einem inerten Verdünnungsmittel statt. Als inerte Verdünnungsmittel eignen sich Wasser, Wasser/Ethanol- oder Wasser/Aceton-Gemische und dergleichen. Bevorzugt wird Wasser eingesetzt. Fumarsäure kann dabei in einer Konzentration bis zur Löslichkeitsgrenze verwendet werden, sodaß entweder eine Lösung oder aber eine Suspension erhalten wird. In diese Lösung bzw. Suspension wird Ammoniak gasförmig, verflüssigt oder in Form einer 10 bis 35 Gew.%igen Lösung eingeleitet, wodurch sich die Temperatur bis auf 60 °C erhöht und sich ein pH-Wert zwischen 7 und 9 einstellt.

Bevorzugt wird eine wässrige 20 bis 30 Gew.%ige Ammoniaklösung verwendet. In das so erhaltene System, vorzugsweise eine Lösung, wird sodann bei 20 bis 60 °C, bevorzugt bei 30 bis 50 °C das Enzym Aspartase oder ein Aspartase-produzierender Mikroorganismus, eingerührt. Bei dieser Zugabe an Enzym- bzw. Aspartase-produzierenden Mikroorganismus ist es von Vorteil, wenn durch die Ammoniakzugabe eine Lösung erhalten wird, da im Falle einer Suspension durch Adsorption des Enzyms und dadurch bedingten Aktivitätsverlust mehr Enzym erforderlich ist. Für einen fast quantitativen Umsatz nach bis zu 24 bis 30 Stunden sind dabei 30 bis 50 IU (Enzymaktivitat) pro Mol Fumarsäure erforderlich.
Aspartase-produzierende Mikroorganismen sind beispielsweise Pseudomonas fluorescens, Protens vulgaris, Pseudomonas aeruginosa, Serratia marcescens, Bacterium succinium, Bacillus subtilis, Aerobacter aerogenes, Micrococcus sp., Escherichia coli u.a.
Weitere geeignete Aspartase-produzierende Mikroorganismen sind beispielsweise in US 3,791,926 und US 3,198,712 beschrieben.
Bei dem erfindungsgemäßen Verfahren kann weiters gereinigte oder synthetische Aspartase eingesetzt werden. Das Enzym bzw. der Aspartase-produzierende Mikroorganismus kann in flüssiger oder in immobilisierter Form, wie beispielsweise in EP 0 127 940 beschrieben, zugesetzt werden.
Nach vollendeter Reaktion, das Reaktionsende kann beispielsweise photometrisch ermittelt werden, erfolgt gemäß Schritt b) gegebenenfalls das Abtrennen von Ammoniak mittels Destillation oder Strippen.

Schritt b) kann bei Normaldruck oder bei reduziertem Druck und bei Temperaturen von 30 bis 110 °C, bevorzugt von 40 bis 90 °C erfolgen.
Überschüssiges Ammoniak wird dabei mittels geläufiger Destillationsmethoden, beispielsweise mittels Kurzweg- oder Dünnschichtverdampfer, Stripper u.s.w., aus dem Reaktionsgemisch entfernt. Je nach Destillationsmethode wird dabei entweder bevorzugt bei Normaldruck oder bei reduziertem Druck zwischen 80 und 200 mbar gearbeitet. Das so erhaltene Ammoniakdestillat wird als Edukt für eine nachfolgende weitere Enzymreaktion a) gemeinsam mit dem Destillat aus dem nachfolgenden Verfahrensschritt d) wiedereingesetzt.
Nach der Ammoniakentfernung oder direkt im Anschluß an Schritt a) erfolgt in Schritt c) das Ausfällen der L-Asparaginsäure.
Dazu wird der Ammonium-L-Aspartat-Lösung soviel Salpetersäure zugegeben, bis ein pH-Wert zwischen 2 und 5 bevorzugt bis der Isoelektrische Punkt erreicht wird. Salpetersäure wird dabei bevorzugt als 10 bis 65 %ige, besonders bevorzugt als 60 %ige Salpetersäure zugesetzt. Die Temperatur des Reaktionsgemisches liegt dabei zwischen 15 und 60 °C, bevorzugt zwischen 20 und 35 °C.

Ab dem 2.ten Reaktionszyklus wird neben der Salpetersäure, das im nachfolgenden Schritt d) erhaltene Retentat aus der Nanofiltration des Fällungsfiltrates, das Restmengen an L-Asparaginsäure und Fumarsäure enthält, der Ammonium-L-Aspartat-Lösung zugegeben.
Anschließend wird das Reaktionsgemisch abgekühlt, bevorzugt auf 0 bis -15 °C und die auskristallisierte L-Asparaginsäure, beispielsweise durch Filtration, wie etwa Absorptionsfiltration, oder durch Zentrifugieren, abgetrennt. Bevorzugt wird L-Asparaginsäure durch Zentrifugation oder Dekantieren abgetrennt.
Die abfiltrierten Kristalle der L-Asparaginsäure werden abschließend gewaschen, vorzugsweise mit Wasser, und getrocknet. Das Waschwasser kann dabei in Schritt a) rückgeführt oder bevorzugt der nachfolgenden Nanofiltration unterzogen werden. Das verbleibende, nach Abtrennung von L-Asparaginsäure erhaltene Fällungfiltrat, sowie das Waschwasser werden sodann in Schritt d) einer Nanofiltration unterzogen. Der pH-Wert liegt dabei bevorzugt zwischen 4 und 11, besonders bevorzugt zwischen 7 und 10, und wird bevorzugt mit NH₃ eingestellt. Der Druck liegt bevorzugt zwischen 1 und 50 bar. Die Temperatur beträgt bevorzugt 10 bis 50 °C, besonders bevorzugt 15 bis 30 °C. Durch die Nanofiltration, kann die in dem Filtrat enthaltene L-Asparaginsäure, sowie Restmengen an Fumarsäure als Ammonsalze, die sich im Retentat befinden aus dem Filtrat zurückgewonnen werden. Das so erhaltene Retentat wird ab dem 2.ten Reaktionszyklus in Schritt c) dem Kristallisationsprozeß zugeführt.
Das durch die Nanofiltration erhaltene Filtrat (ca. 20 %ige Ammonnitratlösung) wird in Schritt e) aufkonzentriert, beispielsweise durch Wasserverdampfung bis eine uber 50 bis 80 %ige, bevorzugt eine etwa 60 bis 70 %ige Ammonnitratlösung erhalten wird. Diese Lösung kann als organisch C-freie Ammonnitratlösung direkt als Wertstoff bzw. Ausgangsstoff in den Stickstoff-Düngeprozeß eingebracht werden.
Das bei der Aufkonzentrierung verbleibende Destillat wird mit dem in Schritt b) gegebenenfalls abgetrennten Ammoniakuberschuß ab dem 2.ten Zyklus, der enzymkatalysierten Reaktion in Schritt a) zugesetzt.

Durch das erfindungsgemäße Verfahren kann L-Asparaginsäure in quantitativen Ausbeuten, d.h. bis zu 99 % und einem Gehalt von >99,5 % erhalten werden.

### Beispiel 1:

In 850 ml (950 g) L-Asparaginsäure-Reaktionslösung, (DSM-Chemie Linz) enthaltend 240 g (1,8 mol) L-Asparaginsäure, hergestellt durch Reaktion aus 210 g) 1,8 mol) Fumarsäure, 200 ml 25 Gew.%ige Ammoniak-Lösung in 280 ml H₂O in Gegenwart von 0,08 ml Aspartase-Lösung (1100 IU/ml), wurde soviel 65 %ige Salpetersäure zugegeben, bis pH 2,7 erreicht wurde (siehe Tabelle 1). Nach 115 Minuten wurde mittels Eis auf 0 °C abgekühlt, der ausgefallene Niederschlag abzentrifugiert, mit 100 ml dest. H₂O nachgewaschen und 10 Minuten trockenzentrifugiert. Es wurden 312 g Feststoff mit einer Feuchte von 22,9 % erhalten. Dies entsprach 225,2 g (93,9 % d.h.) an L-Asparaginsäure trocken.
Weiters wurden 123,7 g (124 ml) Waschwasser und 834,5 g (800 ml) Fällungsfiltrat (Mutterlauge M1) erhalten. Das Waschwasser, sowie M1 wurden mit 25 %igem Ammoniakwasser auf pH9 gestellt und sodann einer Nanofiltration unterzogen. Die so erhaltene etwa 20 % Ammonnitratlösung wurde auf 65 % eingeengt.
Das Retentat wurde bei weiteren Ansätzen in Schritt c) dem Kristallisationsprozeß wieder zugesetzt.

**Tabelle 1:**

| Zeit Min. | g HNO₃ | ml HNO₃ | pH | Temp. |
|---|---|---|---|---|
| 0 | 0 | 0 | 10,2 | 23,4 |
| 10 | 71 | 49 | 9,0 | 30,5 |
| 20 | 88 | 63 | 8,0 | 29,8 |
| 25 | 95 | 65 | 6,0 | 30,0 |
| 35 | 117 | 84 | 5,0 | 30,1 |
| 40 | 135 | 98 | *4,5 auf 5,4 | *30,1 auf 31,3 |
| 55 | 150 | 108 | 5,4 | 30,6 |
| 65 | 186 | 133 | 5,0 | 32,6 |
| 90 | 229 | 166 | 4,5 | 32,2 |
| 100 | 247 | 178 | 4,0 | 32,5 |
| 105 | 255 | 184 | 3,5 | 32,4 |
| 110 | 259 | 186 | 3,0 | 32,2 |
| 115 | 262 | 190 | 2,7 | 31,6 |

| | | | | |
|---|---|---|---|---|
| * Kristallisationsbeginn; kurzfristiger Anstieg des pH-Wertes und der Temperatur | | | | |

## Patentansprüche

1. Verfahren zur Herstellung von L-Asparaginsäure durch enzymkatalysierte Umsetzung von Fumarsäure mit Ammoniak zu Ammonium-L-Aspartat in einem inerten Verdünnungsmittel aus dem nachfolgend die L-Asparaginsäure durch Zusatz einer Säure ausgefällt wird, **dadurch gekennzeichnet, daß**
a) Fumarsäure in einem inerten Verdünnungsmittel in Gegenwart von Aspartase oder Aspartase-produzierenden Mikroorganismen mit Ammoniak zu Ammonium-L-Aspartat umgesetzt,
b) gegebenenfalls überschüssiger Ammoniak aus dem Reaktionsgemisch entfernt und
c) L-Asparaginsäure durch Zugabe von Salpetersäure ausgefällt, aus dem Reaktionsgemisch abgetrennt, gewaschen und getrocknet wird, anschließend
d) aus dem Fällungsfiltrat mittels Nanofiltration Rest-L-Asparaginsäure und Restfumarsäure abgetrennt, das Retentat in Schritt c) rückgeführt wird und
e) das Filtrat aus Schritt d) aufkonzentriert, das dabei anfallende Destillat mit dem in Schritt b) gegebenenfalls abgetrennten Ammoniaküberschuß in Schritt a) wieder zusetzt und gegebenenfalls das aufkonzentrierte Filtrat als Wertstoff für Stickstoffdünger verwendet wird.

2. Verfahren nach Anspruch 1, **dadurch gekennzeichnet, daß** als Verdünnungsmittel in Schritt a) Wasser, Wasser/Ethanol- oder Wasser/Aceton-Gemisch verwendet werden.

3. Verfahren nach Anspruch 1, **dadurch gekennzeichnet, daß** Schritt a) bei einem pH-Wert zwischen 7 und 9 durchgeführt wird.

4. Verfahren nach Anspruch 1, **dadurch gekennzeichnet, daß** gemäß Schritt b) bei Temperaturen zwischen 30 und 110 °C und bei Normaldruck oder bei reduziertem Druck abdestilliert wird.

5. Verfahren nach Anspruch 1, **dadurch gekennzeichnet, daß** Schritt c) bei einem pH-Wert-zwischen 2 und 5 durchgeführt wird.

6. Verfahren nach Anspruch 1, **dadurch gekennzeichnet, daß** Schritt c) bei einer Temperatur zwischen 15 und 60 °C durchgeführt wird.

7. Verfahren nach Anspruch 1, **dadurch gekennzeichnet, daß** die Nanofiltration in Schritt d) bei einem pH-Wert zwischen 4 und 11, einem Druck zwischen 1 und 50 bar und einer Temperatur zwischen 10 und 50 °C durchgeführt wird.

8. Verfahren nach Anspruch 7, **dadurch gekennzeichnet, daß** der pH-Wert zwischen 7 und 10 liegt.

9. Verfahren nach Anspruch 1, **dadurch gekennzeichnet, daß** das Filtrat aus Schritt d) auf eine über 50 bis 80 %ige Lösung aufkonzentriert wird.

## Claims

1. Process for preparing L-aspartic acid by enzyme-catalysed reaction of fumaric acid with ammonia to form ammonia L-aspartate in an inert diluent, from which the L-aspartic acid is subsequently preciptated by addition of an acid, **characterized in that**
a) fumaric acid is reacted with ammonia to form ammonium L-aspartate in the presence of aspartase or aspartase-producing microorganisms in an inert diluent,
b) any excess ammonia is removed from the reaction mixture
and
c) L-aspartic acid is precipitated by adding nitric acid, separated off from the reaction mixture, washed and dried, then
d) residual L-aspartic acid and residual fumaric acid are seperated off from the precipitation filtrate by nanofiltration, the retentate is recycled to step c) and
e) the filtrate from step d) is concentrated, the resultant distillate is added in step a) to any ammonia excess separated off in step b) and if desired the concentrated filtrate is used as a material of value for nitrogen fertilizers.

2. Process according to Claim 1, **characterized in that** the diluent used in step a) is water, water/ethanol or water/acetone mixtures.

3. Process according to Claim 1, **characterized in that** step a) is carried out at a pH between 7 and 9.

4. Process according to Claim 1, **characterized in that**, according to step b), distillation is carried out at temperatures between 30 and 110°C and at atmospheric pressure or under reduced pressure.

5. Process according to Claim 1, **characterized in that** step c) is carried out at a pH between 2 and 5.

6. Process according to Claim 1, **characterized in that** step c) is carried out at a temperature between 15 and 60°C.

7. Process according to Claim 1, **characterized in that** the nanofiltration in step d) is carried out at a pH between 4 and 11, at a prassura between 1 and 50 bar and at a temperature between 10 and 50°C.

8. Process according to Claim 7, **characterized in that** the pH is between 7 and 10.

9. Process according to Claim 1, **characterized in that** the filtrate from step d) is concentrated to an above 50 to 80% strength solution.

## Revendications

1. Procédé de préparation d'acide L-aspartique par réaction, catalysée enzymatiquement, d'acide fumarique avec de l'ammoniac pour obtenir du L-aspartate d'ammonium dans un diluant inerte dans lequel, ensuite, on fait précipiter-l'acide L-aspartique par addition d'un acide, **caractérisé en ce que**
a) on fait réagir de l'acide fumarique dans un diluant inerte en présence d'une aspartase ou de micro-organismes produisant une aspartase avec de l'ammoniac pour obtenir du L-aspartate d'ammonium,
b) le cas échéant, on sépare l'ammoniac en excès du mélange de réaction, et
c) on fait précipiter l'acide L-aspartique par addition d'acide nitrique, on le sépare du mélange de réaction, on le lave et on le sèche et ensuite
d) on sépare l'acide L-aspartique résiduel et l'acide fumarique résiduel du filtrat de précipitation au moyen d'une nanofiltration, on recycle le rétentat dans l'étape c) et
e) on concentre le filtrat de l'étape d), le distillat ainsi obtenu et l'excès d'ammoniac séparé le cas échéant dans l'étape b) sont ajoutés à nouveau dans l'étape a) et, le cas échéant, on utilise le filtrat concentré en tant que matière de valeur pour des engrais azotés.

2. Procédé selon la revendication 1, **caractérisé en ce qu'**on utilise de l'eau, un mélange d'eau/éthanol ou d'eau/acétone en tant que diluant dans l'étape a).

3. Procédé selon la revendication 1, **caractérisé en ce qu'**on effectue l'étape a) à une valeur de pH comprise entre 7 et 9.

4. Procédé selon la revendication 1, **caractérisé en ce que**, conformément à l'étape b), on effectue la séparation par distillation à des températures comprises entre 30 et 110°C et à la pression normale ou à une pression réduite.

5. Procédé selon la revendication 1, **caractérisé en ce qu'**on effectue l'étape c) à une valeur de pH comprise entre 2 et 5.

6. Procédé selon la revendication 1, **caractérisé en ce qu'**on effectue l'étape c) à une température comprise entre 15 et 60°C.

7. Procédé selon la revendication 1, **caractérisé en ce qu'**on effectue la nanofiltration de l'étape d) à une valeur de pH comprise entre 4 et 11, à une pression comprise entre 1 et 50 bars et à une température comprise entre 10 et 50°C.

8. Procédé selon la revendication 7, **caractérisé en ce que** la valeur de pH est comprise entre 7 et 10.

9. Procédé selon la revendication 1, **caractérisé en ce qu'**on concentre le filtrat de l'étape d) de façon à obtenir une solution à une concentration de plus de 50 à 80 %.
